# EUROPÄISCHE PATENTSCHRIFT

(11) **EP 1 926 695 B1**
(45) Veröffentlichungstag und Bekanntmachung des Hinweises auf die Patenterteilung: **15.08.2012**
(21) Anmeldenummer: 06778324.1
(22) Anmeldetag: 23.08.2006
(51) Int. Cl.: C07C 29/17, C07C 29/76, C07C 31/20, C07C 33/035, C07C 33/046

(54) **VERFAHREN ZUR ABTRENNUNG VON POLYMEREN NEBENPRODUKTEN AUS 1,4-BUTINDIOL**
METHOD FOR THE SEPARATION OF POLYMERIC BY-PRODUCTS FROM 1,4-BUTYNEDIOL
PROCEDE DE SEPARATION DE PRODUITS SECONDAIRES POLYMERES CONTENUS DANS LE COMPOSE 1,4-BUTYNDIOL

(30) Priorität: 06.09.2005 DE 102005042184
(43) Veröffentlichungstag der Anmeldung: 04.06.2008
(73) Patentinhaber: BASF SE, 67056 Ludwigshafen (DE)
(72) Erfinder: LORENZ, Rudolf, Erich, 67063 Ludwigshafen (DE); PINKOS, Rolf, 67098 Bad Dürkheim (DE); STEINIGER, Michael, 67435 Neustadt (DE); SCHÄFER, Gerd, 67590 Monsheim (DE); DANNER, Thomas, 67167 Erpolzheim (DE)
(86) Internationale Anmeldenummer: PCT/EP2006/065591
(87) Internationale Veröffentlichungsnummer: WO 2007/028713

(56) Entgegenhaltungen:
- EP-A2- 1 207 146
- DE-A1- 4 432 581
- DE-A1- 19 508 751
- DE-A1- 19 535 450
- DE-C1- 19 625 189
- US-A- 4 294 998

## Beschreibung

Die Erfindung betrifft ein Verfahren zur Reinigung von 1,4-Butindiol durch Abtrennung von Verunreinigungen in Form polymerer Nebenprodukten und von Katalysatorbestandteilen sowie ein Verfahren zur Herstellung von 1,4-Butendiol und 1,4-Butandiol durch Hydrierung des gereinigten 1,4-Butindiols, welches die Abtrennung polymerer Nebenprodukte umfasst.

Die Synthese von 1,4-Butindiol aus Acetylen und Formaldehyd wird industriell mehrfach betrieben und ist beispielsweise bei K. Weissermel, H.-J. Arpe, Industrielle organische Chemie, 5. Auflage, 1998, Wiley-VCH, Seiten 110 und 111) beschrieben worden. Neben Kupfer können die üblicherweise verwendeten Katalysatoren gegebenenfalls Wismut sowie Silikate (im Folgenden SiO₂ genannt) oder Aluminiumoxid enthalten. Während der Synthese von 1,4-Butindiol kommt es als Nebenreaktion zur Bildung von oligomerer beziehungsweise polymerer Substanzen (im Folgenden Cuprene genannt). Diese Cuprene gelangen üblicherweise zusammen mit löslichen und unlöslichen Bestandteilen des verwendeten Katalysators in die Hydrierstufe, bei der zunächst 1,4-Butendiol gebildet wird, das sich in einem weiteren Hydrierschritt zum gegenüber 1,4-Butendiol bedeutsameren Zwischenprodukt 1,4-Butandiol hydrieren lässt.

Die Hydrierung von 1,4-Butindiol zu 1,4-Butandiol wird seit Jahrzehnten betrieben und ist vielfältig beschrieben. So ist aus der US-A 5 068 468 ist die Hydrierung von 1,4-Butandiol an festen geträgerten Nickel-Kupfer-Katalysatoren bekannt, während US-A 4 153 578 ein zweistufiges Verfahren zur Hydrierung von 1,4-Butindiol an suspendierten Raney-Nickel-Molybdän-Katalysatoren bei einem Druck von 21 bar beschreibt. Die DD-A 272 644 offenbart die Suspensionshydrierung von wässrigem Butindiol an Nickel-Si02-Katalysatoren und aus der EP-B 0 319 208, der DE-A 19 41 633 und der DE-A 20 40 501 sind allgemeine, unter anderem auf 1,4-Butindiol anwendbare, Hydrierverfahren bekannt.

Ein Verfahren zur Abtrennung von Feststoffen aus wässriger 1,4-Butindiollösung durch Extraktion mit einem Lösungsmittel in einer Kolonne ist aus DE 195 35 450 bekannt.

Cuprene und Katalysatorbestandteile aus der Butindiolsynthese stören dessen Hydrierung zu 1,4-Butendiol oder 1,4-Butandiol und verschlechtern das Hydrierergebnis deutlich. So lagern sich Cuprene auf dem Katalysator ab und behindern den Kontakt von Butindiol mit der Katalysatoroberfläche mit der Folge, dass die Reaktion langsamer wird. Katalysatorkomponenten wie Kupfer, Wismut und/oder SiO₂ scheiden sich ebenfalls auf Katalysator ab und verändern so die Katalysatoraktivität und Selektivität.

Auch ohne den Ausbau des Katalysators können diese nachteiligen Effekte an der Bildung des Nebenprodukts Butanol verfolgt werden, da dessen Bildung durch die vorstehend genannten Katalysatorgifte beschleunigt wird.

Die einfache Reinigung von 1,4-Butindiol, z.B. durch Filtration ist kaum möglich, da insbesondere die Cuprene und SiO₂ zum Teil kolloidal beziehungsweise feinst verteilt vorliegen und somit gängige Filter schnell verstopfen, so dass die Filter entweder ständig ausgetauscht oder aufwändig rückgespült werden müssen.

Aufgabe der Erfindung ist es, ein einfaches wirtschaftliches Verfahren zur Verfügung zu stellen, mit dem durch die Abtrennung der in der Hydrierstufe unerwünschten Komponenten aus 1,4-Butindiol ein gereinigtes 1,4-Butindiol zugänglich wird, aus dem durch Hydrierung 1.4-Butendiol bzw. 1,4-Butindiol in hoher Selektivität und mit guter Katalysatorstandzeit gewonnen werden kann.

Es wurde nun überraschend gefunden, dass ein Verfahren, bei dem technisches 1,4-Butindiol auf 50 bis 1500 bar komprimiert, entspannt, nach dem Entspannen die Einstellung einer Phasentrennung abgewartet wird und dann die untere Phase abgetrennt wird, zu einer Abtrennung der in der Hydrierstufe unerwünschten Komponenten aus 1,4-Butindiol führt und durch Hydrierung 1,4-Butendiol und 1,4-Butindiol jeweils in hoher Selektivität und mit guter Katalysatorstandzeit zugänglich werden. Unter technischem 1,4-Butindiol wird in dieser Anmeldung aus Acetylen und Formaldehyd an üblichen Katalysatoren hergestelltes 1,4-Butindiol verstanden, das noch nicht beispielsweise durch Destillation gereinigt wurde und im allgemeinen 10 bis 70 Gew.-% Butindiol, bevorzugt 30 bis 60 Gew.-% Butindiol, 0,2 bis 4 Gew.-% Propinol 0,1 - 2 Gew.-% Formaldehyd und 1 bis 2000 ppm, bevorzugt 3 bis 500ppm, besonders bevorzugt 10 bis 200 ppm, Cuprene und Katalysatorbestandteile sowie < 0,1 % andere Verunreinigungen enthält.

1,4-Butindiol wird mit Hilfe handelsüblicher Pumpen auf 50 bis 1500 bar komprimiert. Dabei ist der Erfolg des erfindungsgemäßen Verfahrens um so größer, je höher der Komprimierungsdruck gewählt wird. Bevorzugt liegt dieser Druck über 150 bar, besonders bevorzugt über 250 bar. Die Zeit, in der das 1,4-Butindiol komprimiert vorliegt, ist an sich nicht kritisch. Dieser Zeitraum des Komprimierens kann beispielsweise zwischen 0, 5 Sekunden und 5 Stunden liegen.

Nach dem Komprimieren wird das 1,4-Butindiol entspannt, bevorzugt auf Umgebungsdruck (Normaldruck). Es ist jedoch auch möglich auf Unterdruck , beispielweise im Bereich von 0,5 bar oder auf moderaten Überdruck bis zu 10 bar zu entspannen.

Das Entspannen erfolgt bevorzugt über dem Fachmann bekannte Hochdruckhomogenisiereinrichtungen in Form von Blenden oder Düsen, wie zum Beispiel ein- oder zweistufigen Homogenisierblenden, Flachdüsen oder Microfluidizern, wobei der Einsatz von Lochblenden bevorzugt ist. Wie dem Fachmann bekannt ist, ist die Größe der Lochblende dabei abhängig vom Druck und Durchflussmenge zu wählen. Bei Drucken bis zu 1000bar ist eine Lochblende von 0.4 mm Lochdurchmesser und bei 1000 bis 1500 bar eine Lochblende von 0,2 mm Lochdurchmesser besonders bevorzugt.

Nach dem Entspannen wird die Einstellung einer Phasentrennung abgewartet. Dabei ist es vorteilhaft, das 1,4-Butindiol einer Beruhigungszone zuzuführen. Die Beruhigungszone kann beispielsweise ein Tank oder bevorzugt ein Tank mit konischem Auslauf sein. Die mittlere Verweilzeit des 1,4-Butindiols in der Beruhigungszone, entsprechend der Zeit bis zum Eintritt der Phasentrennung, beträgt im Allgemeinen zwischen einer Stunde und 5 Tagen, bevorzugt zwischen 5 Stunden und 3 Tagen und besonders bevorzugt zwischen 10 Stunden und 30 Stunden.

Während des Lagerns in der Beruhigungszone bilden sich zwei Phasen, die dann mit an sich für diesen Zweck bekannten Verfahren wie z.B. durch Dekantieren oder Zentrifugieren voneinander getrennt werden.

Die schwerere (untere) Phase enthält überwiegend die Cuprene sowie erhebliche Anteile von Kupfer-, Wismut- oder SiO₂- haltigen, unerwünschten Katalysator-Komponenten. Diese untere Phase wird entweder der Entsorgung wie z.B. Verbrennung zugeführt, kann zuvor aber noch z.B. destillativ behandelt werden, um Restanteile an 1,4-Butindiol abzutrennen und ins erfindungsgemäße Verfahren zurückzuführen. Die vom Cuprenen und unerwünschten Katalysatorbestandteilen weitgehend befreite obere Phase wird der Hydrierung zu 1,4-Butendiol oder 1,4-Butandiol zugeführt.

Das erfindungsgemäße Reinigungsverfahren wird im Allgemeinen bei Temperaturen zwischen 10 und 130°C, bevorzugt zwischen 20 und 110°C, besonders bevorzugt zwischen 40 und 100°C durchgeführt und kann absatzweise oder voll kontinuierlich durchgeführt werden, wobei die kontinuierliche Fahrweise bevorzugt ist.

Gegenstand der vorliegenden Erfindung ist zudem ein Verfahren zur Hydrierung von 1,4-Butindiol zu 1,4-Butendiol und bevorzugt zu 1,4-Butandiol, bei dem nach dem erfindungsgemäßen Verfahren gereinigtes 1,4-Butindiol eingesetzt wird.

Die Hydrierung von 1,4-Butindiol ist an sich bekannt und erfolgt bevorzugt in der Flüssigphase an fest angeordneten und/oder suspendierten Katalysatoren. Die Hydrierung kann bis zum 1,4-Butandiol, aber auch nur bis zum 1,4-Butendiol durchgeführt werden.

Für die Hydrierung von gereinigtem 1.4 Butindiol werden solche Katalysatoren verwendet, die C-C-Dreifach- und -Doppelbindungen zu Einfachbindungen zu hydrieren vermögen. Sie enthalten in der Regel eines oder mehrere Elemente der I., VI., VII. oder VIII.-Nebengruppe des Periodensystems der Elemente, bevorzugt die Elemente Kupfer, Chrom, Molybdän, Mangan, Rhenium, Eisen, Ruthenium, Kobalt, Nickel, Platin und Palladium. Besonders bevorzugt verwendet man Katalysatoren, die mindestens ein Element ausgewählt aus Kupfer, Chrom, Molybdän, Eisen, Nickel, Platin und Palladium enthalten.

Der Metallgehalt dieser Katalysatoren liegt in der Regel zwischen 0,1 - 100 Gew.-%, bevorzugt 0,2 - 95 Gew.-%, besonders bevorzugt 0,5 - 95 Gew.-%.

Der Katalysators enthält bevorzugt zusätzlich mindestens ein Element ausgewählt aus den Elementen der II., III., IV. und VI.-Hauptgruppe, der II., III., IV. und V. Nebengruppe des Periodensystems der Elemente und der Lanthanoiden als Promotor zur Aktivitätssteigerung.

Der Promotorgehalt des Katalysators beträgt in der Regel bis 5 Gew.-%, bevorzugt 0,001 - 5 Gew.-%, besonders bevorzugt 0,01 - 3 Gew.-%.

Als Katalysatoren können Fällungs-, Träger-, oder Raney-Typ-Katalysatoren verwendet werden, deren Herstellung beispielsweise in Ullmanns, Encyclopädie der technischen Chemie, 4. Auflage, 1977, Band 13, Seiten 558-665 beschrieben ist.

Als Trägermaterialien können Aluminiumoxide, Titanoxide, Zirkoniumdioxid, Siliciumdioxid, Tonerden, z.B. Montmorillonite, Silikate wie Magnesium- oder Aluminiumsilikate, Zeolithe sowie Aktivkohlen verwendet werden. Bevorzugte Trägermaterialien sind Aluminiumoxide, Titandioxide, Siliciumdioxid, Zirkoniumdioxid und Aktivkohlen. Selbstverständlich können auch Mischungen verschiedener Trägermaterialien als Träger für im erfindungsgemässen Verfahren anwendbare Katalysatoren dienen.

Diese Katalysatoren können entweder als Katalysatorformkörper, beispielsweise als Kugeln, Zylinder, Ringe, Spiralen, oder in Form von Pulvern verwendet werden.

Als Raney-Typ-Katalysatoren sind beispielsweise Raney-Nickel, Raney-Kupfer, Raney-Kobalt, Raney-Nickel/Molybdän, Raney-Nickel/Kupfer, Raney-Nickel/Chrom, Raney-Nickel/Chrom/Eisen oder Rhenium-Schwamm geeignet. Raney-Nickel/Molybdän-Katalysatoren können beispielsweise nach dem in der US-A 4 153 578 beschriebenen Verfahren hergestellt werden. Diese Katalysatoren werden aber auch beispielsweise von der Firma Degussa, 63403 Hanau, Deutschland vertrieben. Ein Raney-NickelChrom-Eisen-Katalysator wird beispielsweise unter der Handelsbezeichnung Katalysator Typ 11 112 W^{®} von der Firma Degussa vertrieben.

Bei der Verwendung von Fäll- oder Trägerkatalysatoren werden diese zu Beginn der Reaktion bei 150 und 500°C im Wasserstoff- bzw. Wasserstoff/Inertgas-Strom reduziert. Diese Reduktion kann direkt im Synthese-Reaktor durchgeführt werden. Falls die Reduktion in einem separaten Reaktor durchgeführt wird, können die Katalysatoren vor dem Ausbau bei 30°C mit sauerstoffhaltigen Gasgemischen oberflächlich passiviert werden. Die passivierten Katalysatoren können in diesem Fall im Synthese-Reaktor vor dem Einsatz in einem Stickstoff/Wasserstoffstrom bei 180°C aktiviert oder auch ohne Aktivierung eingesetzt werden.

Die Katalysatoren können im Festbett oder in Suspension eingesetzt werden. Wenn die Katalysatoren in Form eines Festbetts angeordnet sind, wird der Reaktor nicht in der üblichen Rieselfahrweise, sondern in einem aufwärts gerichteten Gleichstrom von Flüssigkeit und Gas so betrieben, dass die Flüssigkeit und nicht das Gas als kontinuierliche Phase vorliegt.

Suspendierte Katalysatoren werden mit einer Korngröße im Allgemeinen von 0,1 - 500 µm, bevorzugt 0,5 bis 200 µm, besonders bevorzugt 1 bis 100 µm eingesetzt.

Wird mit suspendierten Katalysatoren gearbeitet, so wird bei Verwendung von gepackten Blasensäulen ebenfalls mit einem aufwärts gerichteten Gleichstrom von Flüssigkeit und Gas so gearbeitet, dass die Flüssigkeit und nicht das Gas als kontinuierliche Phase vorliegt. Das Verhältnis von das Reaktionsgefäß verlassender zu zugeführter Gasmenge beträgt bei der Verwendung von Festbettreaktoren und bei der Verwendung von gepackten Blasensäulen mit einem im Reaktionsmedium suspendierten Katalysator 0,99:1 bis 0,4:1.

Das erfindungsgemäß bei Festbettreaktoren und bei im Reaktionsmedium suspendierten Katalysatoren in gepackten Blasensäulen einzuhaltende Verhältnis von das Reaktionsgefäß verlassender zu zugeführter Gasmenge lässt sich in einfacher Weise einstellen, indem man die entsprechende Menge Wasserstoff entweder als Frischgas dosiert, oder technisch bevorzugt, Kreisgas rückführt und nur den durch chemischen Verbrauch und Abgas bedingten Wasserstoffverlust durch Frischwasserstoff ergänzt.

Das molare Verhältnis von Wasserstoff zu 1 ,4-Butindiol im Reaktor beträgt mindestens 3:1,bevorzugt zwischen 4:1 und 100:1.

Das erfindungsgemäße Verfahren wird an Festbettkatalysatoren mit Kreisgasfahrweise durchgeführt, d.h. das den Reaktor verlassende Gas wird im Kreislauf, gegebenenfalls nach Ergänzung mit frischem Wasserstoff, über einen Verdichter in den Reaktor rückgeführt. Es ist möglich, die gesamte Kreisgasmenge oder eine Teilmenge davon über einen Treibstrahlverdichter zu führen. In dieser bevorzugten Ausführungsform wird der Kreisgasverdichter durch eine kostengünstige Düse ersetzt. Die Verdichtungsarbeit wird über die ebenfalls im Kreis geführte Flüssigkeit eingebracht. Die erforderliche Druckerhöhung der Flüssigkeit zum Betreiben des Treibstrahlverdichters beträgt etwa 3 bis 5 bar.

Für die Durchführung des erfindungsgemäßen Verfahrens mit einem im Reaktionsmedium suspendierten Katalysator sind Strahldüsenreaktoren, Rührkessel und Blasensäulen mit Packungen, welche eine Packungsoberfläche von mindestens 500, bevorzugt 1000 bis 2000 m²/m³ aufweisen, geeignet. Strahldüsenreaktoren können in verschiedenen Bauarten Anwendung finden, wenn sie durch einen ausreichend hohen Energieeintrag, der erfahrungsgemäß oberhalb von 2 kW/m³ liegt, den für die Erfindung wesentlichen hohen Stoffübergang von der Gasphase an die Flüssigkeit mit den suspendierten Katalysatorteilchen gewährleisten können. Besonders geeignet sind Strahldüsenreaktoren, die mit einem Impulsaustauschrohr ausgerüstet sind. Eine industriell verbreitete Bauform eines Strahldüsenreaktors ist beispielsweise der in der EP-A 0 419 419 beschriebene Reaktor. Bei Werten für den Energieeintrag von 3 bis 5 kW/m³ ist mit diesem Reaktor eine Abscheidung der Gasphase noch in einfachen Abscheidern möglich, ohne Zusatzapparate wie Schaumzentrifugen oder Zyklone, benutzen zu müssen.

Rührbehälter sind für die Durchführung des erfindungsgemäßen Verfahrens nur dann geeignet, wenn der Energieeintrag in einem Bereich von 2 bis 10 kW/m³ liegt.

Strahldüsenreaktoren mit suspendierten Katalysatoren benötigen volumenbezogene Energieeinträge von mehr als 2 kW/m³, bevorzugt 3 - 5 kW/m³.

1,4-Butandiol findet in der Technik in großen Mengen Anwendung zum Beispiel bei der THF-Herstellung oder als Diolkomponente in Polyestern.

### Beispiele

Das erfindungsgemäße Verfahren wird anhand der nachfolgenden Beispiele näher erläutert. Wenn nicht anders angegeben, wurde technisches 1,4-Butindiol in Form einer 54 gew.-%igen wässrigen Lösung eingesetzt. Die Prozentangaben der Reaktionsausträge in den Beispielen stellen, wenn nicht anders vermerkt, wasserfrei gerechnete Gewichtsprozente dar, die gaschromatographisch ermittelt worden sind.

### Vergleichsbeispiel : technisches 1,4-Butindiol

190 ml eines Ni-Katalysator gemäß Beispiel 1 der US 5 068 468 wurden in einen Hydrierreaktor mit 1,2 m Länge eingefüllt. Technisches 1,4-Butindiol in Form einer 54 gew.-%igen wässrigen Lösung wurde in einer Zulaufmenge von 100g/h in den Reaktor gegeben. Bei einer Temperatur von 140°C wurde bei einem Wasserstoffdruck von 200 bar und einem Flüssigumlauf 800 ml/h wurde 2 Wochen hydriert.

Innerhalb des Versuchszeitraums (2 Wochen) war der Butindiolumsatz immer vollständig. Die durchschnittliche Zunahme an n-Butanol lag bei ca. 0,06 % pro Tag. Nach Ausbau des Katalysators fanden sich Feststoffablagerungen auf dem Katalysator. Die Ausbeute an 1,4-Butandiol, bezogen auf eingesetztes Butindiol, zu Beginn 98,3 Gew. %, verringerte sich jedoch jeden Tag um 0,06 -Gew.-%.

### Beispiel 1: Reinigung des 1,4-Butindiols

### Reinigung:

Technisches Butindiol der im Vergleichsversuch genannten Zusammensetzung wurde mit einer Hochdruckpumpe auf 1000 bar bei 50°C bis zu einer Sekunde komprimiert und anschließend sofort über die Lochblende (0,2 mm Durchmesser) entspannt. Nach dem Entspannen über die Lochblende wurde das so gereinigte Butindiol dem Hydrierreaktor zugeführt.

### Hydrierung:

Die Hydrierung wurde analog dem Vergleichsbeispiel durchgeführt, mit dem Unterschied, dass das erfindungsgemäß vorgereinigtes 1,4-Butindiol eingesetzt wurde. Die durchschnittliche Zunahme an n-Butanol lag bei ca. nur 0,04 % pro Tag. Nach Ausbau des Katalysators fanden sich keine Ablagerungen. Die Ausbeute an 1,4-Butandiol, bezogen auf eingesetztes Butindiol, betrug zu Beginn 98,3 Gew.-% und verringerte sich jeden Tag um 0,04 Gew.-% %.

## Patentansprüche

1. Verfahren zur Reinigung 1,4-Butindiol, **dadurch gekennzeichnet, dass** 1,4-Butindiol auf 50 bis 1500 bar komprimiert, entspannt, nach dem Entspannen die Einstellung einer Phasentrennung abgewartet und die untere Phase abgetrennt wird.

2. Verfahren nach Anspruch 1, **dadurch gekennzeichnet, dass** auf 150 bis 1500 bar komprimiert wird.

3. Verfahren nach Anspruch 1 oder 2, **dadurch gekennzeichnet, dass** bei 10 bis 130°C komprimiert wird.

4. Verfahren nach einem der Ansprüche 1 bis 3, **dadurch gekennzeichnet, dass** 0,5 Sekunden bis 5 Stunden komprimiert wird.

5. Verfahren nach einem der Ansprüche 1 bis 4 , **dadurch gekennzeichnet, dass** über eine Homogenierungseinrichtung, ausgewählt aus ein- oder zweistufigen Homogenisierblenden, Flachdüsen oder Microfluidizern, entspannt wird.

6. Verfahren nach einem der Ansprüche 1 bis 5, **dadurch gekennzeichnet, dass** auf Umgebungsdruck entspannt wird.

7. Verfahren nach einem der Ansprüche 1 bis 6, **dadurch gekennzeichnet, dass** die Einstellung der Phasentrennung in einer Beruhigungszone erfolgt.

8. Verfahren nach einem der Ansprüche 1 bis 7, **dadurch gekennzeichnet, dass** aus der abgetrennten unteren Phase 1,4-Butindiol gewonnen und in das Reinigungsverfahren zurückgeführt wird.

9. Verfahren zur Herstellung von Butendiol durch katalytische Hydrierung von 1,4-Butindiol, **dadurch gekennzeichnet dass** nach dem Verfahren nach einem der Ansprüche 1 bis 7 gereinigtes 1,4-Butindiol hydriert wird.

10. Verfahren zur Herstellung von Butandiol durch katalytische Hydrierung von 1,4-Butindiol, **dadurch gekennzeichnet dass** nach dem Verfahren nach einem der Ansprüche 1 bis 7 gereinigtes 1,4-Butindiol hydriert wird.

## Claims

1. A process for purifying 1,4-butynediol, which comprises compressing 1,4-butynediol to from 50 to 1500 bar, depressurizing it, waiting for phase separation to occur after depressurization and separating off the bottom phase.

2. The process according to claim 1, wherein the 1,4-butynediol is compressed to from 150 to 1500 bar.

3. The process according to claim 1 or 2, wherein compression is carried out at from 10 to 130°C.

4. The process according to any of claims 1 to 3, wherein the 1,4-butynediol is compressed for from 0.5 seconds to 5 hours.

5. The process according to any of claims 1 to 4, wherein the 1,4-butynediol is depressurized through a homogenization device selected from among single-stage or two-stage orifice homogenizers, flat nozzles and microfluidizers.

6. The process according to any of claims 1 to 5, wherein the 1,4-butynediol is depressurized to ambient pressure.

7. The process according to any of claims 1 to 6, wherein phase separation occurs in a calming zone.

8. The process according to any of claims 1 to 7, wherein 1,4-butynediol is recovered from the bottom phase which has been separated off and is recirculated to the purification process.

9. A process for preparing butenediol by catalytic hydrogenation of 1,4-butynediol, wherein 1,4-butynediol purified by the process according to any of claims 1 to 7 is hydrogenated.

10. A process for preparing butanediol by catalytic hydrogenation of 1,4-butynediol, wherein 1,4-butynediol purified by the process according to any of claims 1 to 7 is hydrogenated.

## Revendications

1. Procédé de purification de 1,4-butynediol, **caractérisé en ce que** le 1,4-butynediol est comprimé à une pression de 50 à 1 500 bar, détendu, l'ajustement d'une séparation de phases est attendu après la détente et la phase inférieure est séparée.

2. Procédé selon la revendication 1, **caractérisé en ce que** la compression est réalisée à une pression de 150 à 1 500 bar.

3. Procédé selon la revendication 1 ou 2, **caractérisé en ce que** la compression est réalisée à une température de 10 à 130 °C.

4. Procédé selon l'une quelconque des revendications 1 à 3, **caractérisé en ce que** la compression est réalisée pendant 0,5 seconde à 5 heures.

5. Procédé selon l'une quelconque des revendications 1 à 4, **caractérisé en ce que** la détente est réalisée par un dispositif d'homogénéisation choisi parmi des déflecteurs d'homogénéisation à un ou deux niveaux, des buses plates ou des microfluidiseurs.

6. Procédé selon l'une quelconque des revendications 1 à 5, **caractérisé en ce que** la détente est réalisée à la pression ambiante.

7. Procédé selon l'une quelconque des revendications 1 à 6, **caractérisé en ce que** l'ajustement de la séparation de phases a lieu dans une zone de repos.

8. Procédé selon l'une quelconque des revendications 1 à 7, **caractérisé en ce que** le 1,4-butynediol est récupéré à partir de la phase inférieure séparée et recyclé dans le procédé de purification.

9. Procédé de fabrication de butènediol par hydrogénation catalytique de 1,4-butynediol, **caractérisé en ce que** du 1,4-butynediol purifié par un procédé selon l'une quelconque des revendications 1 à 7 est hydrogéné.

10. Procédé de fabrication de butanediol par hydrogénation catalytique de 1,4-butynediol, **caractérisé en ce que** du 1,4-butynediol purifié par un procédé selon l'une quelconque des revendications 1 à 7 est hydrogéné.
